## Europäisches Patentamt
## European Patent Office
## Office européen des brevets

(11) Veröffentlichungsnummer: **0 053 579**
**B1**

(12) **EUROPÄISCHE PATENTSCHRIFT**

(45) Veröffentlichungstag der Patentschrift:
02.10.85

(21) Anmeldenummer: 81810436.6

(22) Anmeldetag: 30.10.81

(51) Int. Cl.⁴: **A 61 B 6/00,** H 02 K 41/02,
G 03 B 42/04

(54) Röntgenanlage.

(30) Priorität: 27.11.80 CH 8796/80

(43) Veröffentlichungstag der Anmeldung:
09.06.82 Patentblatt 82/23

(45) Bekanntmachung des Hinweises auf die Patenterteilung:
02.10.85 Patentblatt 85/40

(84) Benannte Vertragsstaaten:
DE FR GB IT NL SE

(56) Entgegenhaltungen:
DE - A - 1 466 880
DE - B - 1 029 121
DE - B - 1 112 887
DE - B - 1 192 364
DE - C - 949 980
US - A - 2 182 985
US - A - 3 175 085
US - A - 3 891 856
US - A - 3 986 697

(73) Patentinhaber: RHYN, MECHANISCHE WERKSTATT,
Industriestrasse, CH-4573 Lohn (CH)

(72) Erfinder: Rhyn, Werner, Solothurnstrasse 108,
CH-3349 Kräiligen (CH)

(74) Vertreter: Seehof, Michel et al, c/o AMMANN
PATENTANWAELTE AG BERN Schwarztorstrasse 31,
CH-3001 Bern (CH)

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

## Beschreibung

Die vorliegende Erfindung bezieht sich auf eine Röntgenanlage mit einem Stativ, bestehend aus einer Säule mit einem U-förmigen Profil und einem daran angeordneten, höhenverstellbaren, gleitbar befestigten Schlitten, an welchem über eine Drehscheibe und einen Zapfen ein Querbalken befestigt ist, an dessen beiden Enden ein Röntgengenerator bzw. ein Röntgenfilmhalter angeordnet sind.

Eine solche Röntgenanlage ist in der DE-B-1 192 364 beschrieben. Der innerhalb der Säule angeordnete Schlitten wird mittels Gewichtsausgleich über einen Seilzugmechanismus bewegt, wobei es vorkommen kann, daß das oder die Seile aus den Führungsrollen springt und es zu Verklemmungen kommt und außerdem ist mit solchen Verstellmechanismen oft eine starke Geräuschentwicklung verbunden.

Aus der DE-B-1 029 121 ist eine Einrichtung zum Antrieb des Wagens eines Röntgengerätes bekannt, in welcher ein elektrischer Linearantrieb verwendet wird. Der Antrieb arbeitet mit einem Gewichtsausgleichsystem mit Kabel zusammen und weist auch die oben angegebenen Nachteile auf und dient zudem nur als Unterstützung des Handbetriebes. Außerdem ist die Läuferstange starr an der Säule befestigt, wodurch sie genauestens justiert werden muß, um zufriedenstellend zu laufen.

Schließlich werden bekannte Röntgenanlagen in der Regel jeweils für einen bestimmten Anwendungszweck hergestellt, wodurch sowohl der Hersteller als auch der Verkäufer gezwungen sind, eine große Typenvielfalt auf Lager zu halten.

Es ist demgegenüber Aufgabe der vorliegenden Erfindung eine Röntgenanlage anzugeben, die einen leichtgängigen, geräuscharmen und genauen Verstellmechanismus aufweist und modular aufgebaut ist, um für verschiedene Anwendungszwecke leicht nach dem Baukastenprinzip zusammengesetzt zu werden.

Diese Aufgabe wird mit einer in den Ansprüchen beschriebenen Röntgenanlage gelöst.

Die Erfindung wird nun im einzelnen anhand einer Zeichnung von Ausführungsbeispielen näher erläutert werden.

Fig. 1 zeigt eine räumliche Ansicht von vorne des Stativs der erfindungsgemäßen Röntgenanlage.

Fig. 2 zeigt einen Aufriß der Säule von hinten gemäß Fig. 1.

Fig. 3 zeigt eine Seitenansicht der Säule von Fig. 2.

Fig. 4 zeigt in größerem Maßstab eine Draufsicht, teilweise geschnitten, auf die Säule von Fig. 2.

Fig. 5 zeigt eine Draufsicht der Befestigung des Querbalkens an der Säule.

Fig. 6 zeigt einen Schnitt der Befestigung von Fig. 5.

Fig. 7 zeigt eine Ausführungsvariante der Befestigung des Querbalkens an die Säule.

Fig. 8 zeigt einen Schnitt der Befestigung von Fig. 7.

Fig. 9 zeigt schematisch die Verwendung einer Röntgenanlage mit einem Röntgenfilmhalter-Wandstativ.

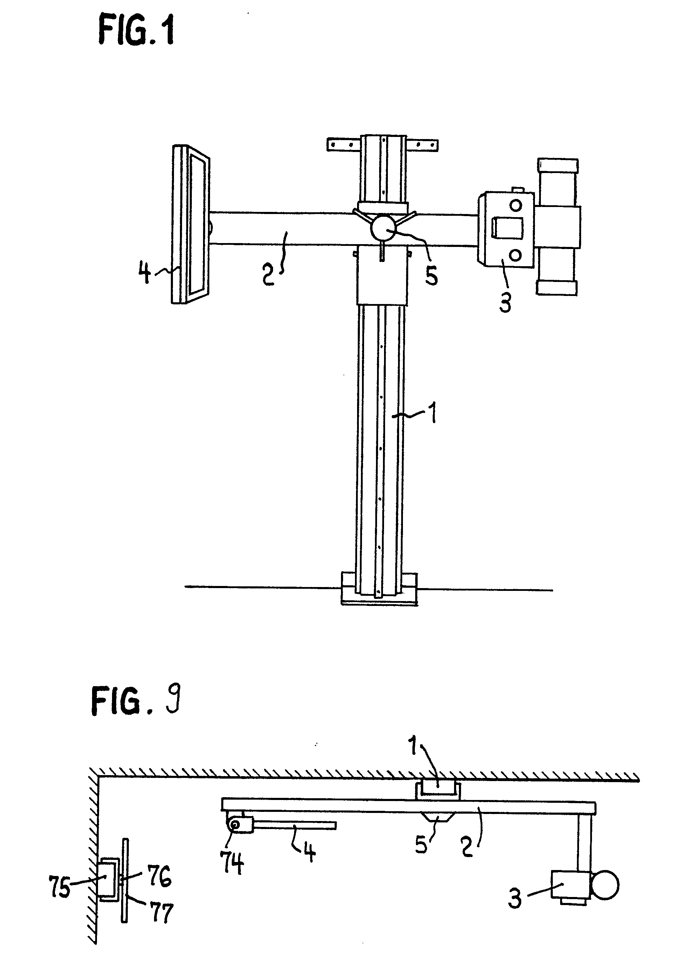

Man erkennt in Fig. 1 die hauptsächlichsten Elemente einer Röntgenanlage, das aus der Säule 1 und dem Querbalken 2 bestehende Stativ, wobei an einem Ende des Balkens der Röntgengenerator 3 und am anderen Ende der Röntgenfilmhalter 4 befestigt ist. Der Querbalken ist mit Befestigungsmitteln 5, die weiter unten eingehender beschrieben werden, derart an der Säule befestigt, daß er entweder stufenweise oder stufenlos verschwenkt werden kann. Erforderlichenfalls kann der Abstand zwischen der Filmkassette und dem Röntgengenerator durch nicht dargestellte Mittel verändert werden.

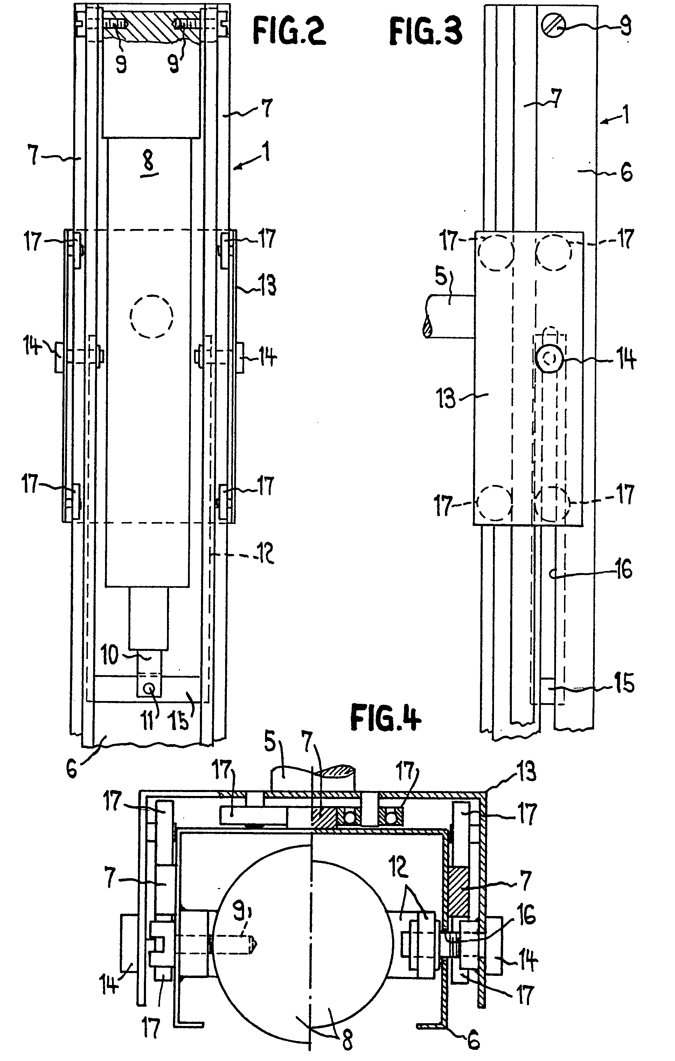

In den Fig. 2 bis 4 sind die Mittel zur Höhenverstellung des Querbalkens dargestellt. Die Säule 1 besteht aus einem U-förmigen Profil 6, das an der Wand befestigt werden kann und das an seinen drei Frontseiten je eine Führungsschiene 7 aufweist. Innerhalb dieses U-förmigen Profils ist ein elektrischer Linearantrieb 8 mittels zweier Schrauben 9 aufgehängt. Dieser Linearantrieb ist vorzugsweise selbsthemmend und in beiden Richtungen stufenlos verstellbar ausgebildet. Die Schubstange 10 des Linearantriebs ist mittels eines Bolzens 11 mit einem Bügel 12 verbunden, der an einem Schlitten 13 befestigt ist. Der Schlitten 13 ist in seiner Mitte mittels Schrauben 14 mit den beiden Enden des Bügels 12 verbunden, während die Schubstange 10 am Quersteg 15 des Bügels angreift. Um die Verschiebung des Schlittens 13 zu ermöglichen, ist in den Seitenwänden des U-förmigen Profils 6 je ein Schlitz 16 angeordnet. Der Schlitten 13 wird mittels beidseitig den Führungsschienen 7 angeordneten Kugellagerpaaren 17 geführt. Aus obiger Beschreibung geht hervor, daß die Montage und Justierung des Linearantriebes sehr einfach und exakt mittels der Schrauben 9 erfolgen kann, während der Schlitten, an welchem der Querbalken mit den Instrumenten an den Befestigungsmitteln 5 befestigt werden kann, eine gute Führung aufweist und mit geringem Kraftaufwand auf- und niederbewegt werden kann.

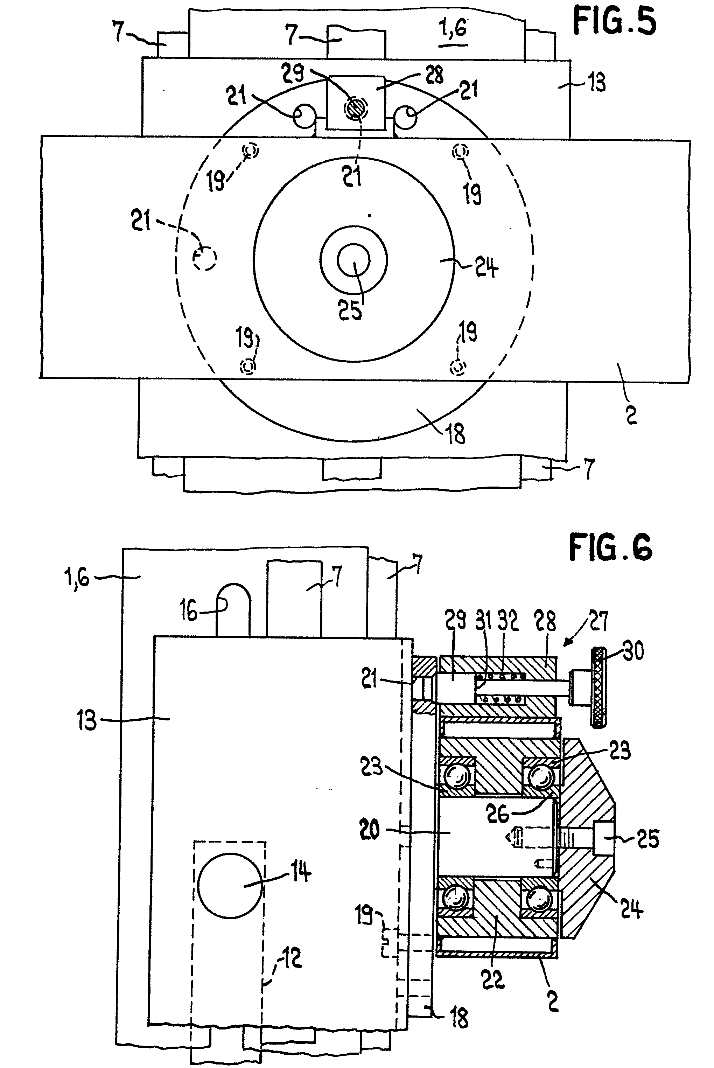

Röntgenanlagen werden, je nach Einsatzgebiet und Verwendungszweck, in den verschiedensten Ausführungen benötigt, und es ist daher sowohl für den Hersteller als auch für den Verkäufer und Monteur vorteilhaft, wenn nur wenig verschiedene Teile vorhanden sind, welche sich leicht kombinieren lassen. Während die Säule mit dem Linearantrieb in sämtlichen Ausführungen die gleiche bleibt, ist es vorteilhaft, den Querbalken oder dessen Längsverstellbarkeit oder seine Verschwenkbarkeit den vorgegebenen Verhältnissen anzupassen und gegebenenfalls den Röntgenfilmhalter derart zu gestalten, daß Kassetten mit verschiedenen Formaten verwendet werden können. Dabei spielen die Befestigungsmittel eine große Rolle, die einerseits eine leichte Austauschbarkeit der Querbalken und andererseits eine bequeme und zuverlässige Verschwenkung, wie beispielsweise stetig oder stufenweise, ermöglichen sollen. In den Fig. 5 und 6 ist eine Ausführungsform solcher Befestigungsmittel dargestellt. Auf der Frontseite des Schlittens 13 ist eine Scheibe 18 mittels Schrauben 19 angeflanscht, wobei diese Scheibe einen Zapfen 20 trägt. In der oberen Hälfte der Scheibe 18 sind, wie aus Fig. 5 hervorgeht, Bohrungen 21 angebracht, in welche ein Stift eines Einrastmechanismus einrastet. Im Zentrum des Querbalkens 2 ist ein Kugellagergehäuse 22 angeschweißt, in welchem zwei Kugellager 23 angeordnet sind. Über dem Gehäuse 22 ist eine Verschlußkappe 24 angeordnet, die mittels eines Schraubenbolzens 25 am Zapfen 20 befestigt wird. Die Unterseite der Verschlußkappe 24 weist an der dem Zapfen 20 gegenüberliegenden Stelle einen Absatz 26 auf, während das obere Kugellager leicht aus dem Gehäuse 22 herausragt, so daß beim Befestigen der Verschlußkappe 24 ein Druck auf das obere Kugellager ausgeübt wird, um eine Vorspannung desselben zu bewirken. Diese Vorspannung bewirkt einen Widerstand, so daß der Querbalken nicht frei beweglich ist und beim Auslösen des Einrastmechanismus von alleine in Rotation gerät. Der Einrastmechanismus 27 besteht aus einem am Querbalken befestigten Gehäuse 28, einem in die Öffnungen 21 hineinpassenden Stift 29 mit einem Betätigungsknopf 30 und einem Absatz 31, auf den eine Druckfeder 32 abgestützt ist, um nach dem Herausziehen und Loslassen des Stiftes diesen wieder in die gewählte Öffnung 21 zu drücken. Aus obiger Beschreibung dieser Befestigungsmittel geht hervor, daß einerseits ein Querbalken leicht ausgewechselt und befestigt werden kann, da dieser nur auf den Zapfen 20 gesteckt und befestigt werden muß und bereits einen großen Halt aufweist, und daß andererseits der Querbalken sehr leicht durch Herausziehen des Stiftes 29 in die jeweils gewünschten Positionen geschwenkt werden kann.

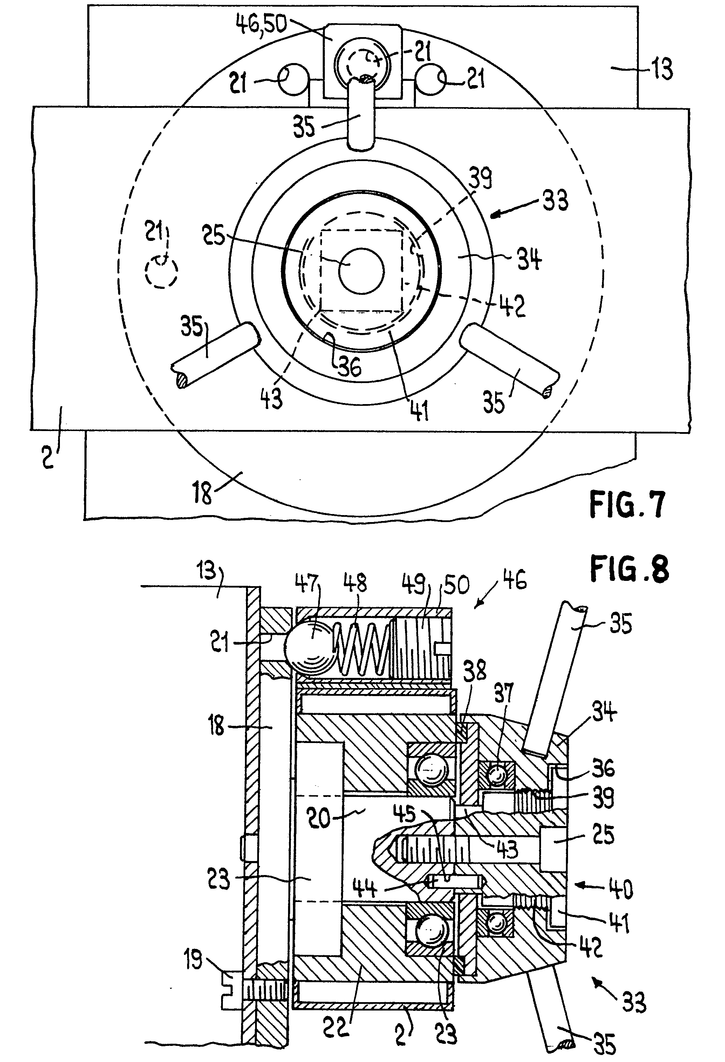

In den Fig. 7 und 8 ist eine Ausführungsvariante einer Verschlußkappe dargestellt, mit welcher es möglich ist, den Querarm kontinuierlich zu verschwenken und zu fixieren. Die Scheibe 18 mit den Rastbohrungen 21 und dem Zapfen 20 ist die gleiche wie im vorhergehenden Beispiel, ebenso wie das Kugellagergehäuse 22 mit den Kugellagern 23. Die Verschlußkappe 33 besteht im wesentlichen aus zwei gegeneinander verschiebbaren Teile, wobei am Außenteil 34 Drehgriffe 35, beispielsweise drei, befestigt sind. Das Außenteil 34 weist an seiner Stirnseite inneren eine Ausnehmung 36, daran anschließend ein Innengewinde 39 und ein Kugellager 37, daran anschließend eine Scheibe mit einer Dichtung 38 auf, wobei die Innenseite der Scheibe einen quadratischen Querschnitt aufweist. Das Außenteil 34 der Verschlußkappe wird durch ein Innenteil 40 gesichert, das mittels des Schraubenbolzens 25 mit dem Zapfen 23 verbunden ist. Das Innenteil 40 weist an seiner Stirnseite eine in die Ausnehmung 36 passende Schulter 41 auf, an die sich ein Zapfen anschließt, der von der Schulter aus gesehen ein zylindrisches Teil mit einem Gewinde 42, ein zylindrisches Teil ohne Gewinde und ein Teil mit quadratischem Querschnitt 43 aufweist, das in die Scheibe mit Dichtung paßt, sowie einen Stift 44, der an der inneren Stirnseite des Innenteils angeordnet ist und in eine entsprechende Bohrung 45 im Schaft 23 greift. Dabei sind die Ausnehmung 36 und die Dicke der Scheibe 41 derart gewählt, daß das Außenteil 34 einen gewissen Spielraum hat, um das Drehen desselben zu ermöglichen, wodurch der Querbalken freigegeben wird und verschwenkt werden kann. Durch diese Konstruktion ist es möglich, durch eine kleine Drehung am Außenteil 34 bzw. an den Griffen 35 den Querbalken verstellen zu können, ohne daß jedoch dieser frei drehen kann. Außerdem ist ein am Querbalken 2 befestigter Einrastmechanismus 46 vorgesehen, der eine Kugel 47, eine Druckfeder 48 und eine Befestigungsschraube 49 aufweist und in einem Gehäuse 50 angeordnet ist. Auch bei dieser Konstruktion ist das Auswechseln des Querbalkens mit den daran befestigten Apparaturen sehr einfach.

In Fig. 9 ist die Verwendung einer erfindungsgemäßen Röntgenanlage für die Thorax-Radiographie schematisch angegeben, wofür man zwischen Röntgengenerator und Röntgenplatte bekanntlich einen Abstand von zwei Metern benötigt. Bei dieser Anlage ist die Röntgenfilmhalter-Befestigung 74 schwenkbar, so daß der Halter, wie in der Fig. 9 eingezeichnet, zum Querarm hin geklappt werden kann. Als zweite Säule kann entweder Säule 1 oder eine vereinfachte Säule 75 mit einer Führungsschiene 76 verwendet werden, auf der eine vereinfachte Röntgenfilm-Halterung 77 verstellbar angeordnet ist. Durch die Anordnung gemäß Fig. 12 kann eine raumsparende Anlage geschaffen werden, die sämtliche Röntgenaufnahmen ermöglicht.

**Patentansprüche**

1. Röntgenanlage mit einem Stativ, bestehend aus einer Säule (1) mit einem U-förmigen Profil (6) und einem daran angeordneten, höhenverstellbaren, gleitbar befestigten Schlitten (13), an welchem über eine Drehscheibe (18) und einen Zapfen (20) ein Querbalken (2) befestigt ist, an dessen beiden Enden ein Röntgengenerator (3) bzw. ein Röntgenfilmhalter (4) angeordnet sind, dadurch gekennzeichnet, daß der Schlitten (13) außen am U-förmigen Profil (6) gleitet und zur Verschiebung des Schlittens (13) innerhalb des U-förmigen Profils (6) ein elektrischer Linearantrieb (8) hängend befestigt ist, dessen Schubstange (10) an dem Quersteg (15) eines Bügels (12) befestigt ist, der seinerseits am Schlitten befestigt ist.

2. Röntgenanlage nach Anspruch 1, dadurch gekennzeichnet, daß der elektrische Linearantrieb (8) selbsthemmend und in beide Richtungen stufenlos verstellbar ausgebildet ist.

3. Röntgenanlage nach Anspruch 1 oder 2, dadurch gekennzeichnet, daß das U-förmige Profil (6) an seinen drei Stirnseiten je eine Führungsschiene (7) aufweist und der Schlitten (13) innen mit drei Kugellagerpaaren (17) versehen ist, die jeweils beidseitig der Führungsschienen angreifen.

4. Röntgenanlage nach einem der Ansprüche 1 bis 3, dadurch gekennzeichnet, daß der Querbalken (2) in seinem Zentrum ein um den Zapfen (20) der Säule passendes, zwei Kugellager (23) aufweisendes Kugellagergehäuse (22) aufweist und eine Verschlußkappe (24), die mittels eines Schraubenbolzens (25) mit dem Zapfen (20) verbunden ist, wobei der dem Zapfen gegenüberliegende Teil der Unterseite der Verschlußkappe abgesetzt (26) ist, derart, daß der übrige Teil der Verschlußkappe das obere Kugellager vorspannt, wodurch der Querbalken mit Widerstand drehbar ist.

5. Röntgenanlage nach Anspruch 4, dadurch gekennzeichnet, daß am Querbalken ein Einrastmechanismus (27) angeordnet ist, mit einem federbelasteten Stift (29), der in eines der in der Drehscheibe (18) angeordneten Rastlöcher greift.

6. Röntgenanlage nach Anspruch 4, dadurch gekennzeichnet, daß die Verschlußkappe (33) zweiteilig ausgeführt ist, mit einem Außenteil (34), an dem Drehgriffe (35) befestigt sind und das an der Stirnseite eine Ausnehmung (36) und anschließend ein Innengewinde (39) und an der inneren Stirnseite eine Scheibe mit einer Dichtung (38) und einer quadratischen Öffnung aufweist und einem Innenteil (40), mit einer in die Ausnehmung passenden Schulter (41), einem Zapfen mit Gewinde (42) und einem Teil (43) mit quadratischem Querschnitt, wobei der Querbalken einen Einrastmechanismus (46) aufweist.

7. Verwendung der Röntgenanlage nach einem der Ansprüche 1 bis 6 für die Thoraxradiographie, dadurch gekennzeichnet, daß der Röntgenfilmhalter (4) schwenkbar am Querarm befestigt ist und daß in einem bestimmten Abstand vom Röntgengenerator (3) eine zweite Säule (75) angeordnet ist, an welcher ein zweiter Röntgenfilmhalter (77) verschiebbar befestigt ist.

## Claims

1. X-ray apparatus with a support, consisting of a column (1) with a U-shaped profile (6) supporting a vertically adjustable, slidably mounted carriage (13), to which is secured by means of a rotatable disc (18) and a journal (20) a transverse beam (2), to the ends of which are respectively disposed a X-ray generator (3) and a X-ray film holder (4), characterized in that the carriage (13) slides outside of the U-shaped profile (6) and in that for shifting the carriage (13), an electrical linear drive (8) is suspended in the inside of the U-shaped profile (6), the push rod (10) of the drive being secured to the cross-bar (15) of a clamp (12) which is itself secured to the carriage.

2. X-ray apparatus according to claim 1, characterized in that the electrical linear drive (8) is self-blocking and continuously adjustable in both directions.

3. X-ray apparatus according to claim 1 or 2, characterized in that the U-shaped profile (6) comprises at each of its three front faces a guide rail (7), and in that the carriage (13) is provided at the inside with three pairs of ball-bearings (17) which engage respectively on both sides of the guide rail.

4. X-ray apparatus according to one of the claims 1 to 3, characterized in that the transverse beam (2) comprises in its centre a ball-bearing housing (22) comprising two ball-bearings (23) and fitted to the journal (20) of the column and a cover cap (24) connected by a stud (25) to the journal (20), the lower part of the cover cap facing the journal being offset, so that the remaining part of the cover cap biases a tension on the upper ball bearing whereby the transverse beam is rotatable with an impediment.

5. X-ray apparatus according to claim 4, characterized in that a locking device (27) is mounted to the transverse beam and comprises a springloaded pin (29) which engages in one of the stop holes of the rotatable disc (18).

6. X-ray apparatus according to claim 4, characterized in that the cover cap (33) is in two parts, with an external part (34) to which are secured rotary handles (35) and comprising at its front face a recess (36) and adjacent an internal threading (39) and at its inner front face a disc with a packing (38) and a square aperture and an internal part (40) with a shouler (41) and a part (43) with a square cross-section, the transverse beam comprising a locking device (46).

7. Utilization of the X-ray apparatus according to one of the claims 1 to 6 for radiography of the thorax, characterized in that the X-ray film holder (4) is pivotably secured to the transverse beam and in that at a determined distance from the X-ray generator (3) is provided a second column (75) to which is displaceably secured a second X-ray film holder (77).

## Revendications

1. Appareil de radiologie avec un support, consistant en une colonne (1) avec un profil en U (6) sur lequel est disposé un chariot (13) fixé de manière coulissante et déplaçable en hauteur, auquel est fixée par un disque tournant (18) et un tenon (20) une traverse (2) aux extrémités de laquelle sont disposés un générateur à rayons X (3), respectivement un porte-film Röntgen (4), caractérisé en ce que le chariot (13) coulisse à l'extérieur du profil en U (6) et que pour déplacer le chariot (13), un entraînement linéaire électrique (8) est suspendu à l'intérieur du profil en U (6), la tige de poussée de l'entraînement étant fixée à la branche transversale (15) d'un étrier (12) fixé lui-même au chariot.

2. Appareil de radiologie selon la revendication 1, caractérisé en ce que l'entraînement linéaire électrique (8) est autobloquant et déplaçable sans gradations dans les deux directions.

3. Appareil de radiologie selon la revendication 1 ou 2, caractérisé en ce que le profil en U (6) comprend à chacune de ses trois faces frontales un rail de guidage (7) et en ce que le chariot (13) est pourvu à l'intérieur de trois paires de roulements à billes (17) qui s'engagent respectivement des deux côtés des rails de guidage.

4. Appareil de radiologie selon l'une des revendications 1 à 3, caractérisé en ce que la traverse (2) comprend en son centre un logement de roulements à billes (22) comprenant deux roulements à billes (23) et adapté au tenon (20) de la colonne et un chapeau de fermeture (24) connecté par un boulon fileté (25) au tenon (20), la partie inférieure du chapeau de fermeture située en face du tenon étant dégagée, de sorte que la partie restante du chapeau de fermeture exerce une précontrainte sur le roulement à billes supérieur, ce qui permet une rotation de la traverse avec une résistance.

5. Appareil de radiologie selon la revendication 4, caractérisé en ce qu'un mécanisme à encliquetage (27) est monté à la traverse et comprend une cheville chargée par un ressort (29) qui s'engage dans un des trous d'arrêt du disque tournant (18).

6. Appareil de radiologie selon la revendication 4, caractérisé en ce que le chapeau de fermeture (33) est en deux parties, avec une partie extérieure (34) à laquelle sont fixées des poignées de rotation (35) et comprenant dans sa face frontale un dégagement (36) et un filetage intérieur adjacent (39) et à sa face frontale intérieure un disque avec un joint (38) et une ouverture quadratique, et une partie intérieure (40) avec un collier (41) adapté dans le dégagement, un tenon fileté (42) et une partie (43) avec une section quadratique, la traverse comportant un mécanisme d'encliquetage (46).

7. Utilisation de l'appareil de radiologie selon l'une des revendications 1 à 6 pour la radiographie du thorax, caractérisée en ce que le porte-film Röntgen (4) est fixé de manière pivotante à la traverse et en ce qu'à une distance déterminée du générateur de rayons X (3) est disposée une deuxième colonne (75) à laquelle est fixé de manière déplaçable un deuxième porte-film Röntgen (77).

**FIG.1**

**FIG. 9**

0 053 579

FIG.2  FIG.3

FIG.4

0 053 579

FIG.5

FIG.6

FIG.7

FIG.8